# EUROPEAN PATENT SPECIFICATION

(11) **EP 1 030 638 B1**
(45) Date of publication and mention of the grant of the patent: **12.03.2003**
(21) Application number: 98959530.1
(22) Date of filing: 20.11.1998
(51) Int. Cl.: A61F 13/15, A61F 15/00

(54) **PLURALITY OF ABSORBENT INTERLABIAL PRODUCTS DISPENSER**
SPENDER FÜR INTERLABIALE DAMENBINDEN
DISTRIBUTEUR POUR PLUSIEURS PRODUITS ABSORBANTS A PLACER EN POSITION INTERLABIALE

(30) Priority: 21.11.1997 US 975795; 18.03.1998 US 40599
(43) Date of publication of application: 30.08.2000
(73) Proprietor: THE PROCTER & GAMBLE COMPANY, Cincinnati, Ohio 45202 (US)
(72) Inventor: OSBORN, Thomas, Ward, III, Cincinnati, OH 45220 (US); JOHNSON, Matthew, Jeremiah, Waseca, MN 56093 (US); TWEDDELL, Richard, III, Cincinnati, OH 45229 (US)
(74) Representative: Kremer, Véronique
(86) International application number: US9824810
(87) International publication number: WO99026576

(56) References cited:
- GB-A- 2 316 618
- US-A- 4 564 108

## Description

This invention relates to packages for absorbent devices that are worn interlabially by female wearers for catamenial purposes, incontinence protection, or both. More particularly, the present invention relates to dispensers for interlabial devices that provide ease of use and convenience of transporting and dispensing more than one of the interlabial devices.

### BACKGROUND OF THE INVENTION

All manner and variety of absorbent articles configured for the absorption of body fluids such as menses, urine and feces are, of course, well known. With respect to feminine protection devices, the art has offered two basic types; sanitary napkins have been developed for external wear about the pudendal region while tampons have been developed for internal wear within the vaginal cavity for interruption of menstrual flow therefrom.

Hybrid devices which attempt to merge the structural features of the sanitary napkins and the tampons into a single device have also been proposed. Such hybrid devices are disclosed in U.S. Patent No. 2,092,346, entitled "Catamenial Pad", issued to Arone on September 7, 1937, and U.S. Patent No. 3,905,372, entitled "Feminine Hygiene Protective Shield", issued to Denkinger on September 16, 1975. Other less intrusive hybrid devices are known as labial or interlabial sanitary napkins and are characterized by having a portion which at least partially resides within the wearer's vestibule and may also have a portion which at least partially resides external of the wearer's vestibule. Such devices are disclosed in U.S. Patent No. 2,662,527, entitled "Sanitary Pad", issued to Jacks on December 15, 1953, and U.S. Patent No. 4,631,062, entitled "Labial Sanitary Pad", issued to Lassen, et al. on December 23, 1986.

Interlabial pads have the potential to provide even greater freedom from inconvenience because of their small size and reduced risk of leakage. Numerous attempts have been made in the past to produce an interlabial pad which would combine the best features of tampons and sanitary napkins while avoiding at least some of the disadvantages associated with each of these types of devices. Examples of such devices are described in U.S. Patent 2,917,049 issued to Delaney on December 15, 1959, U.S. Patent 3,420,235 issued to Harmon on January 7, 1969, and U.S. Patent 4,595,392 issued to Johnson, et al. on June 17, 1986. A commercially available interlabial device is FRESH 'N FIT® PADETTE® interlabial product which is marketed by Athena Medical Corp. of Portland, OR and described in U.S. Patents 3,983,873 and 4,175,561 issued to Hirschman on October 5, 1976 and November 27, 1979, respectively.

If the consumer needs only one interiabia pad for later use, the consumer must take precautions to protect the interlabial pad from soiling or contamination from the time it is removed from the box or bag until the article is used. This is a particular concern with respect to maintaining a sanitary environment during insertion and removal. That is, a need exists to hygienically store an individual interlabial pad while being transported to prevent transferring unsanitary particles to the interlabial space.

Packages for sanitary napkins are known, for example in US Patent No. 4,564,108 a package in which a plurality of napkins are wrapped in a sealed packaging is disclosed.

Interlabial devices are typically changed every time a woman urinates since the product will generally occlude both the urethreal and vaginal openings. Due to the high frequency of urination most women experience, it is a distinct advantage to be able to easily and discreetly carry multiple interlabial products throughout the day in a package where they are individually wrapped for hygiene reasons, but as a composite package for ease of use and convenience.

### SUMMARY OF THE INVENTION

This invention relates to packages for absorbent devices that are worn interlabially by female wearers for catamenial purposes, incontinence protection, or both. More particularly, the present invention relates to dispensers for interlabial devices that provide ease of use and convenience of transporting and dispensing more than one of the interlabial devices.

The package for a plurality of absorbent interiabial products comprises a continuous strip of connected product comprising two or more packages for the absorbent interlabial products. The packages have seals that form an individual and separate inner compartment to protect the absorbent interlabial products from contaminants. The packages are joined together by a frangible connection located in between or around seals that provides a line of weakness, that when broken, separates the packages into individual packages of absorbent interlabial products.

In one preferred embodiment, the package for a plurality of absorbent interlabial products also comprises a case. The case can be in any suitable configuration. In one embodiment, the case has an upper wall, lower wall, and at least one side wall oriented longitudinally between the upper wall and the lower wall. The side wall is attached along the outer perimeter of both the upper wall and the lower wall oppositely connecting the upper and the lower wall to form an enclosed shape. The side wall has an opening from which the packages are dispensed from the interior of the case.

### BRIEF DESCRIPTION OF THE DRAWINGS

While the specification concludes with claims particularly pointing out and distinctly claiming the subject matter which is regarded as forming the present invention, it is believed that the invention will be better understood from the following description taken in conjunction with the accompanying drawings, in which:
FIG. 1 is a perspective view of one embodiment of an absorbent interlabial device.
FIG. 2 is an end view of the absorbent device shown in FIG. 1.
FIG. 3 is a perspective view of another embodiment of an absorbent interlabial device.
FIG. 4 is a side view of an individually packaged absorbent interlabial device.
FIG. 5 is a perspective view of one embodiment of a case of a plurality of absorbent interlabial devices.
FIG. 6 is a perspective view of an alternate embodiment of a case of a plurality of absorbent interlabial devices.
FIG. 7 is a plan view of a series of horizontally connected packages of individual absorbent interlabial devices.
FIG. 8 is a plan view of a series of longitudinally connected packages of individual absorbent interlabial devices.
FIG. 9 is a side view one embodiment of an individual container for packaging absorbent interlabial devices or individually packaged interlabial devices.

### DETAILED DESCRIPTION OF THE INVENTION

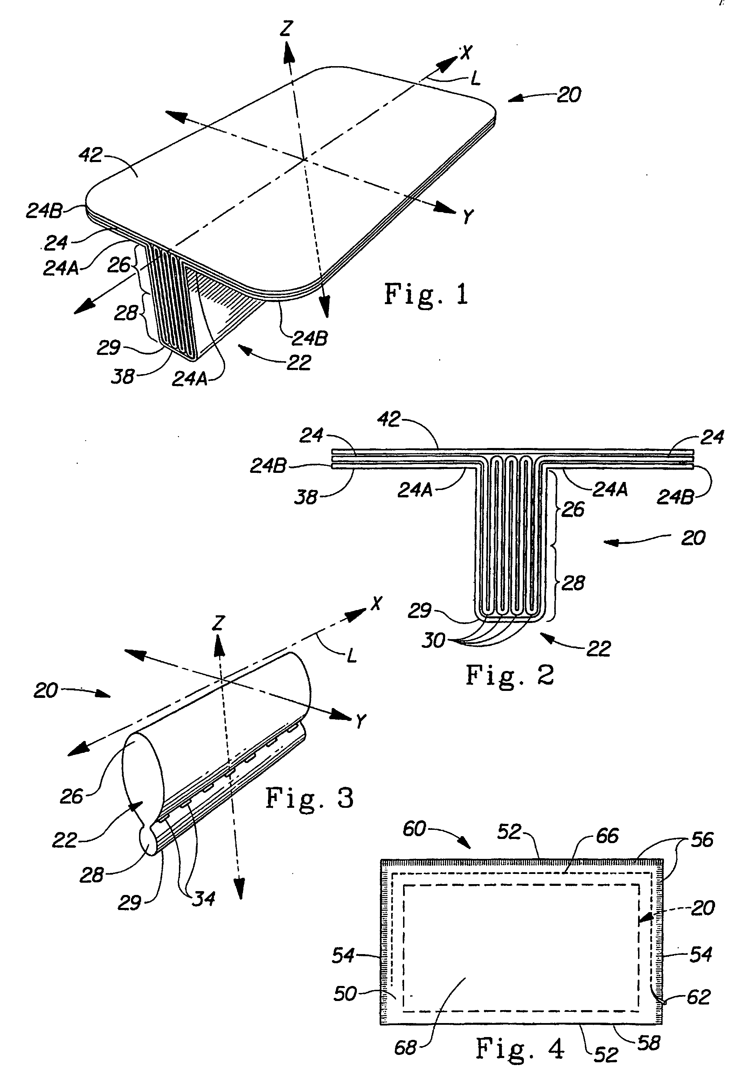

The present invention is directed to an individual package for an absorbent interlabial device. FIG. 1 shows one preferred embodiment of the absorbent interlabial device 20. The interlabial device, however, can be in many other forms, and is not limited to a structure having the particular configuration shown in the drawings.

As used herein, the term "absorbent interlabial device" refers to a structure which has at least some absorbent components, and is specifically configured to reside at least partially within the interlabial space of a female wearer during use. Preferably, more than half of the entire absorbent interlabial device 20 resides within such interlabial space, more preferably substantially the entire absorbent interlabial device 20 resides within such interlabial space, arid most preferably the entire absorbent interlabial device 20 resides within such interlabial space of a female wearer during use.

As used herein, the term "interlabial space" refers to that space in the pudendal region of the female anatomy which is located between the inside surfaces of the labia majora extending into the vestibule. Located within this interlabial space are the labia minor, the vestibule and the principal urogenital members including the clitoris, the orifice of the urethra, and the orifice of the vagina. Standard medical authorities teach that the vestibule refers to the space bounded laterally by the inside surfaces of the labia minora and extending interiorly to the floor between the clitoris and the orifice of the vagina. Therefore, it will be recognized that the interlabial space as defined above may refer to the space between the inside surfaces of the labia majora, including the space between the inside surfaces of the labia minora also known as the vestibule. The interlabial space for purposes of the present description does not extend substantially beyond the orifice of the vagina into the vaginal interior.

The term "labia" as used herein refers generally to both the labia majora and labia minora. The labia terminate anteriorly and posteriorly at the anterior commissure and the posterior commissure, respectively. It will be recognized by those skilled in the art that there is a wide range of variation among women with respect to the relative size and shape of labia majora and labial minora. For purposes of the present description, however, such differences need not be specifically addressed. It will be recognized that the disposition of the absorbent interlabial device into the interlabial space of a wearer as defined above will require placement between the inside surfaces of the labia majora without regard to the precise location of the boundary between the labia majora and the labia minora for a particular wearer. For a more detailed description of this portion of the female anatomy, attention is directed to *Gray's Anatomy,* Running Press 1901 Ed. (1974), at 1025-1027.

The absorbent interlabial device 20 shown in FIG. 1 has a longitudinal centerline L which runs along the "x" axis shown in FIG. 1. The term "longitudinal", as used herein, refers to a line, axis or direction in the plane of the interlabial device 20 that is generally aligned with (e.g., approximately parallel to) a vertical plane which bisects a standing wearer into left and right body halves and extends between the front and rear of the wearer's body when the interlabial device 20 is worn. The terms "transverse" and "lateral" as used herein, are interchangeable, and refer to a line axis or direction that is generally perpendicular to the longitudinal direction (that is, outward from this vertical plane toward the wearer's thighs). The lateral direction is shown in FIG. 1 as the "y" axis. The "z" direction, shown in FIG. 1, is a direction parallel to the vertical plane described above. The term "upper" refers to an orientation in the z-direction toward the wearer's head. "Lower" or downwardly is toward the wearer's feet.

In the embodiment shown in FIG. 1, the interlabial device 20 comprises a central absorbent portion (or "main absorbent") 22, and optionally, a pair of flexible extensions 24 joined to the central absorbent portion 22. The central absorbent portion 22 should be at least partially absorbent. The central absorbent portion 22 may comprise non-absorbent portions, such as a liquid impervious barrier to prevent absorbed exudates from leaking out of the central absorbent portion 22. The central absorbent portion 22 comprises an upper portion 26 and a lower portion 28 that is opposed to the upper portion. As shown in FIGS. 1 and 2, where the central absorbent portion 22 is of a uniform transverse dimension (i.e., there is no abrupt change in transverse dimension defining the juncture between the upper portion and lower portion) the division between the upper portion 26 and lower portion 28 is considered to be at a height equal to about one-half of the total height of the central absorbent portion 22. The flexible extensions 24 are joined to the upper portion 26 of the central absorbent portion. In use, the upper portion 26 is positioned furthest inward into the wearer's interlabial space. The lower portion 28 has a base (or "bottom edge" or "lower edge") 29.

The interlabial device 20 should be of a suitable size and shape that allows at least a portion thereof to fit comfortably within the wearer's interlabial space and to cover the wearer's vaginal orifice, and preferably also the wearer's urethra. The interlabial device 20 at least partially blocks, and more preferably completely blocks and intercepts the flow of menses, urine, and other bodily exudates from the wearer's vaginal orifice and urethra.

The size of the interlabial device 20 is also important to the comfort associated with wearing the device. In the embodiment shown in FIGS. 1, 2 and 3, the width of the central absorbent portion 22 of the interlabial device 20 as measured in the transverse direction (y-direction) is preferably between about 2 mm and less than or equal to about 12 mm, more preferably between about 3 mm and about 8 mm. In a preferred embodiment, the width of the central absorbent portion of the interlabial device 20 is about 4.5 mm. The central absorbent portion 22 of the interlabial device 20 has a length as measured along the longitudinal centerline, L, of between about 35 mm and about 120 mm. Preferably, the length of the interlabial device 20 is between about 45 mm and about 100 mm, and more preferably, is about 49-85 mm. The height (or "z"-direction dimension) of the central absorbent portion 22 is preferably between about 8 mm and about 35 mm, and more preferably is about 20 mm. Caliper measurements given herein were measured using an AMES gage with a 0.25 psi (gauge) load and a 0.96 inch diameter foot. Those skilled in the art will recognize that if a 0.96 inch diameter foot is not appropriate for a particular sample size, the foot size may be varied while the load on the gauge is accordingly varied to maintain a confining pressure of 0.25 psi (gauge).

The interlabial device 20 is preferably provided with sufficient absorbency to absorb and retain the exudates discharged from the wearer's body. The capacity of the product, however, is dependent at least partially upon the physical volume of the absorbent interlabial device 20, particularly the central absorbent portion 22 thereof. The central absorbent portion 22 preferably has a capacity of at least about 1 g of 0.9% by weight saline solution, and may have a capacity of up to about 30 g by using absorbent gels or foams that expand when wet. Capacities may typically range from about 2 to about 10 grams, for saline. Those skilled in the art will recognize that the capacity for absorption of body exudates such as menses will typically be smaller than the capacities given above for absorption of saline. A method for measuring absorbent capacity is described in the Test Methods section, below. Since the interlabial space can expand, larger volumes can be stored in the interlabial space, if the fluid is stored as a gel, which adjusts to the body pressures. Additionally, if the absorbent interlabial device 20 does not reside completely within the wearer's interlabial space, some of the absorbed exudates may be stored externally to the wearer's interlabial space.

The central absorbent portion 22 of the interlabial device 20 may comprise any suitable type of absorbent structure that is capable of absorbing and/or retaining liquids (e.g. menses and/or urine). The central absorbent portion 22 may be manufactured in a wide variety of shapes. Non-limiting examples include ovoid, trapezoidal, rectangular, triangular, cylindrical, hemispherical or any combination of the above. The central absorbent portion 22 may, likewise, be manufactured and from a wide variety of liquid-absorbent materials commonly used in absorbent articles such as comminuted wood pulp which is generally referred to as airfelt. Examples of other suitable absorbent materials include cotton; creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these. Preferred absorbent materials comprise folded tissues, woven materials, nonwoven webs, needle punched rayon, and thin layers of foam. The central absorbent portion 22 may comprise a single material or a combination of materials, such as a wrapping layer surrounding a central wadding comprised of a different absorbent material.

In the embodiment shown in FIGS. 1 and 2, the central absorbent portion 22 of the absorbent interlabial device 20 comprises a pleated structure. As shown in FIGS. 1 and 2, the central absorbent portion 22 comprises a folded tissue web. The folded tissue web preferably has a strength greater than that of standard non-wet strength toilet tissue. Preferably, the central absorbent portion 22 comprises a tissue having a temporary wet strength of greater than or equal to about 100 g. In a preferred embodiment, this wet strength will decay to about 50% or less of the original strength over about 30 minutes when measured under a wet burst strength test.

As shown in FIGS. 1 and 2, the tissue web comprising the central absorbent portion 22 is folded into a pleated structure comprising a plurality of pleats 30 that are arranged in a laterally side-by-side relationship. The tissue web can be folded so that it has any suitable number of pleats. Preferably, the tissue web is folded so that the overall caliper (i.e., the width) of the central absorbent portion 22 of this embodiment is between about 2 mm and less than or equal to about 8 mm.

The pleats in the folded tissue web are preferably connected or joined (or retained) in some suitable manner so that the pleated sections maintain their pleated configuration, and are not able to fully open. The pleats can be connected by a variety of means including the use of thread, adhesives, or heat sealing tissues which contain a thermoplastic material, such as, polyethylene. A preferred design uses stitching which joins all of the pleats in the central absorbent portion 22 together with cotton fiber. Preferably, the main absorbent structure 22 is provided with five stitch locations (four at the comers and one additional location approximately midway between the two lower comers).

The pleated structure of the central absorbent portion 22 provides several advantages. One advantage provided by the pleated structure is that exudates can penetrate into the pleats of the structure which present a larger and more effective absorbent surface for acquisition than a flat surface. This is particularly important when dealing with potentially viscous fluids and particulate material such as cellular debris and clots which can plug the surface of the structure presented to the body. A second advantage of this design is that the caliper (or width) of the product can be easily and conveniently controlled by varying the number of pleats. Another advantage of the pleated structure is that the number, thickness, and tightness of the pleats control the stiffness of the structure.

The stiffness of the central absorbent portion 22 is important for product comfort. If the central absorbent portion 22 is too flexible, the device is not conveniently or easily placed between the folds of the labia, if it is too stiff, the device is uncomfortable and when the user is in a sitting position, the product can be forced forward against the clitoris causing discomfort. The central absorbent portion 22 preferably has a stiffness approximately equal to that of the products described in US Patents 4,995,150 and 4,095,542.

In the embodiment shown in FIG. 3, the central absorbent portion 22 is formed of a soft absorbent material such as rayon fibers or other suitable natural or synthetic fibers or sheeting. The central absorbent portion 22 shown in FIG. 3 is generally of an ovoid cross sectional shape. The central absorbent portion 22 of the embodiment shown in FIG. 3 comprises an upper portion 26 with a larger transverse sectional dimension relative to that of the lower portion 28. The upper portion 26 is preferably integral with the lower portion 28. In less preferred embodiments, however, the upper portion 26 and lower portion 28 may comprise separate elements joined together by any suitable means know in the art. In the embodiment shown in FIG. 3, the juncture of the upper portion 26 and lower portion 28 of the central absorbent portion 22 comprises a substantially abrupt change in the transverse dimension thereby forming a shoulder-like configuration at such juncture. In the embodiment shown in FIG. 3, the juncture of the upper portion 26 and lower portion 28 of the central absorbent portion 22 is formed by stitching 34.

In a variation of the embodiment described above and shown in FIG. 3, the upper portion 26 may have a smaller transverse sectional dimension relative to the transverse sectional dimension of the lower portion 28, and the portions may have different absorbent capacities. In other embodiments, such as that shown in FIGS. 1 and 2, the juncture between the upper portion 26 and the lower portion 28 can be indistinguishable.

The central absorbent portion 22 can be made by any suitable process. US Patent 4,995,150 issued to Gerstenberger et al. on February 26, 1991 and US Patent 4,095, 542 issued to Hirschman on June 20, 1978 describe methods for making absorbent devices which are suitable for use as the central absorbent portion 22 of the absorbent interlabial device 20 shown in FIG. 3.

As shown in FIGS. 1 and 2, the absorbent interlabial device 20 preferably also comprises a pair of flexible extensions 24 which are joined to the upper portion 26 of the central absorbent portion 22 of the absorbent interlabial device 20. In the preferred embodiment shown in FIGS. 1 and 2, the flexible extensions 24 are generally rectangular in shape. Other shapes are also possible for the flexible extensions 24 such as semi-circular, trapezoidal, or triangular. The flexible extensions 24 preferably are from about 40 mm to about 160 mm in length, more preferably from about 45 mm to about 130 mm in length, and most preferably from about 50 mm to about 115 mm in length. While the flexible extensions 24 can have a length (measured in the x-direction) which is shorter than the central absorbent portion 22, preferably they have a length which is the same as or longer than the central absorbent portion 22 of the absorbent interlabial device 20. The width of each flexible extensions refers to the distance from the attachment of flexible extension 24 to the central absorbent portion 22 (or the proximal end 24A of the flexible extension 24) to the distal end (or free end) 24B of the flexible extension 24. The width of the flexible extensions 24 is preferably about equal to or greater than the height of the central absorbent portion as described above. The caliper of the flexible extensions is preferably less than or equal to about 3 mm, more preferably less than or equal to about 2 mm, and most preferably less than or equal to about 1 mm. Ideally, the caliper of the flexible extensions 24 and the central absorbent portion 22 are selected such that the caliper of the overall absorbent interlabial structure 20 is less than or equal to about 8 mm.

The flexible extensions 24 may be constructed of a tissue layer. A suitable tissue is an airlaid tissue available from Fort Howard Tissue Company of Green Bay, Wisconsin, and having a basis weight of 35 lbs./3000 sq. ft. Another suitable airlaid tissue is available from Merfin Hygienic Products, Ltd., of Delta, British Columbia, Canada, having a basis weight of 61 g/m² and having the designation grade number 176. Still another suitable material is an airlaid cotton batt such as that sold as cosmetic squares by Revco stores, Inc. of Twinsberg, OH. The flexible extensions 24 may optionally be backed with a layer of material which is impervious or semi-pervious to body exudates such as polyethylene, polypropylene, or a polyvinylalchohol.

In the embodiment shown in FIGS. 1 and 2, the pair of flexible extensions 24 may comprise a single sheet of material extending to either side of the longitudinal centerline L of the central absorbent portion 22 of the absorbent interlabial device 20. Alternatively, the pair of flexible extensions 24 may comprise separate sheets of material independently joined to the upper portion 26 of the central absorbent portion 22. Preferably, the flexible extensions 24 are arranged symmetrically about the longitudinal centerline L of the central absorbent portion 22. The flexible extensions 24 are joined to the upper portion 26 of the central absorbent portion 22 of the absorbent interlabial device 20. Most preferably, the flexible extensions are joined to the top surface of the upper portion 26 of the central absorbent portion 22, or within about 5 mm of the top surface of the central absorbent portion 22.

The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element; i.e., one element is essentially part of the other element.

As shown in FIGS. 1 and 2, the flexible extensions 24 are attached to the upper portion 26 of the central absorbent portion 22. The flexible extensions 24 extend downwardly and outwardly from the central absorbent portion 22 to a free end 24B which is unattached to the central absorbent portion. The flexible extensions 24 may be biased slightly outward from the central absorbent portion 22 so as to tend to keep the extensions 24 in contact with the inner surfaces of the labia when the absorbent interlabial device 20 is in place. Additionally, the naturally moist surfaces of the labia will have a tendency to adhere to the material comprising the flexible extensions 24 further tending to keep them in contact with the inner surfaces of the labia. Preferably, the flexible extensions 24 should be capable of motion from a position where the free ends of the flexible extensions 24 lie adjacent to the central absorbent portion 22 to a position where the flexible extensions 24 extend directly out from the central absorbent portion 22 in the transverse direction.

The flexible extensions 24 may be joined to the upper portion 26 of the central absorbent portion 22 by any variety of means. For example, in the embodiment shown in FIGS. 1 and 2, flexible extensions 24 may be joined to the upper portion 26 using any suitable adhesive centered about the longitudinal centerline L of the central absorbent portion 22 (i.e., on opposite sides of the longitudinal centerline L). The adhesive may extend continuously along the length of the central absorbent portion 22 or it may be applied in a "dotted" fashion at discrete intervals. Alternatively, the flexible extensions 24 may be joined to the upper portion 26 of the central absorbent portion 22 by stitching (such as with cotton or rayon thread), thermally bonding, fusion bonding, or any other suitable means known in the art for joining such materials.

The flexible extensions 24 should be of sufficient width and flexibility to allow the flexible extensions to cover the wearer's fingertips as the absorbent interlabial device 20 is inserted into the wearer's interlabial space. Additionally, the flexible extensions 24 should be capable of moving with the inner surfaces of the wearer's labia to maintain contact with the same. The flexible extensions 24 help keep the central absorbent portion 22 in place throughout a range of wearer motions such as squatting.

The flexible extensions 24 may be hydrophilic or hydrophobic. The flexible extensions 24 may be treated to make them less hydrophilic than the central absorbent portion 22. The hydrophilicity of a material is generally expressed in terms of its contact angle. Thus, the flexible extensions 24 may have an advancing contact angle greater than the advancing contact angle of the central absorbent portion 22, such that fluid is preferentially directed toward and absorbed by the central absorbent portion 22. The flexible extensions 24 may be either absorbent or non-absorbent. Preferably, the flexible extensions 24 have at least some absorbency. However, the majority of the fluid absorbed and retained by the absorbent interlabial device 20 will preferably ultimately be retained in the central absorbent portion 22. For a more detailed description of hydrophilicity and contact angles see the following publications which are incorporated by reference herein: The American Chemical Society Publication entitled "Contact Angle, Wettability, and Adhesion," edited by Robert F. Gould, and copyrighted in 1964; and TRI/Princeton Publications, Publication Number 459, entitled "A Microtechnique for Determining Surface Tension," published in April 1992, and Publication Number 468 entitled, "Determining Contact Angles Within Porous Networks," published in January, 1993, both edited by Dr. H. G. Heilweil.

The pleated design shown in FIGS. 1 and 2 has the additional benefit of easily providing the flexible extensions 24. The extensions 24 can comprise the same material as the central absorbent portion 22, or they can comprise a different material. The extensions 24 are joined to the upper portion 26 of the central absorbent portion 22, and most preferably, for this embodiment, are joined to the top surface of the central absorbent portion 22, or within 1 millimeter of the top surface of the central absorbent portion 22. Preferably, in the embodiment shown in FIGS. 1 and 2, the extensions 24 are integral portions of the central absorbent portion 22 (that is, the extensions 24 comprise integral extensions of the absorbent tissue material that is folded to form the central absorbent portion 22.

The interlabial device 20 in any of the embodiments shown in the drawings may comprise other optional components. For example, the interlabial device 20 may comprise a topsheet 42 positioned over and joined to all or a portion of the body facing surface of the device 20 and/or a backsheet 38 positioned over and joined to all or a portion of its back surface, including the flexible extensions 24. Preferably, if a topsheet 42 and/or a backsheet 38 is used, these components are joined to at least a portion of the central absorbent portion. In an alternative embodiment, the central absorbent portion could be at least partially wrapped by a topsheet 42.

If a topsheet is used, the topsheet should be compliant, soft feeling, and nonirritating to the wearer's skin. Further, the topsheet should be liquid pervious permitting liquids (e.g., menses and/or urine) to readily penetrate through its thickness. A suitable topsheet may be manufactured from a wide range of materials such as woven and nonwoven materials comprised of natural fibers (e.g., wood or cotton fibers), synthetic fibers (e.g., polymeric fibers such as polyester, rayon, polypropylene, or polyethylene fibers) or from a combination of natural and synthetic fibers. Other woven and nonwoven materials may include polymeric materials such as apertured formed thermoplastic films, apertured plastic films, and hydroformed thermoplastic films; porous foams; reticulated foams; reticulated thermoplastic films; and thermoplastic scrims.

The topsheet may comprise an apertured formed film. Apertured formed films are pervious to body exudates and, if properly apertured, have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent 3,929,135, entitled "Absorptive Structures Having Tapered Capillaries", which issued to Thompson on December 30, 1975; U.S. Patent 4,324,246 entitled "Disposable Absorbent Article Having A Stain Resistant Topsheet", which issued to Mullane, et al. on April 13, 1982; U.S. Patent 4,342,314 entitled "Resilient Plastic Web Exhibiting Fiber-Like Properties", which issued to Radel, et al. on August 3, 1982; U.S. Patent 4,463,045 entitled "Macroscopically Expanded Three-Dimensional Plastic Web Exhibiting Non-Glossy Visible Surface and Cloth-Like Tactile Impression", which issued to Ahr, et al. on July 31, 1984; and U.S. 5,006,394 "Multilayer Polymeric Film" issued to Baird on April 9, 1991. The preferred topsheet for the interlabial device is the formed film described in one or more of the above patents and marketed on sanitary napkins by The Procter & Gamble Company of Cincinnati, Ohio as the "DRI-WEAVE" topsheet.

If such a formed film is used in an interlabial device, the body surface of the formed film topsheet is preferably hydrophilic so as to help liquid to transfer through the topsheet faster than if the body surface was not hydrophilic so as to diminish the likelihood that menstrual fluid will flow off the topsheet rather than flowing into and being absorbed by the central absorbent portion 22. In a preferred embodiment, surfactant is incorporated into the polymeric materials of the formed film topsheet. Alternatively, the body surface of the topsheet can be made hydrophilic by treating it with a surfactant such as is described in U.S. Patent 4,950,254 issued to Osborn.

If a backsheet is used, the backsheet 38 could be impervious or semi-pervious to liquids (e.g., menses and/or urine) and is preferably flexible. As used herein, the term "flexible" refers to materials which are compliant and will readily conform to the general shape and contours of the human body. The backsheet 38 prevents the exudates absorbed and contained in the central absorbent portion 22 from wetting articles which contact the absorbent interlabial device 20 such as the wearer's undergarments. The backsheet also assists the central absorbent portion 22 in preventing the wearer's body from being soiled by exudates. Additionally, use of the backsheet may provide an improved surface for the wearer to grasp between the fingers as the absorbent interlabial device 20 is inserted, or as the device is optionally removed with the fingers.

The backsheet 38 may comprise a woven or nonwoven material, polymeric films such as thermoplastic films of polyethylene or polypropylene, or composite materials such as a film-coated nonwoven material. Preferably, the backsheet 38 is a film having a thickness of from about 0.012 mm (0.5 mil) to about 0.051 mm (2.0 mils). An exemplary polyethylene film is manufactured by Clopay Corporation of Cincinnati, Ohio, under the designation P18-0401. The backsheet may permit vapors to escape from the central absorbent portion 22 (i.e., breathable) while still preventing exudates from passing through the backsheet.

The embodiments in FIGS. 1-3 may be packaged in an individual package 60 as shown in FIG. 4. The individual package 60 comprises a wrapper 50 that encloses the absorbent interlabial device 20 to provide a sanitary environment. The wrapper 50 should at least partially enclose the absorbent interlabial device 20, and preferably completely encloses the absorbent interlabial device 20. The wrapper 50 preferably comprises a rectangular sheet of flexible material. The wrapper 50 can be folded about or wrapped around the absorbent interlabial device 20 in any suitable manner.

The individual package 60 preferably has perforations 66 that are positioned along the upper longitudinal edge 52 and extend substantially along each side edge 54 of the package. The individual package 60 preferably also has a tear strip or string 62 that generally extends along and in the direction of the perforations 66. The individual package 60 is opened by using the tear string 62 to break the perforations 66 along a significant portion of the periphery of the individual package 60. This releases two distinct side panels 68 which will drape over both sides of the user's fingers, and exposes the absorbent interlabial device 20. In some alternate embodiments, the tear strip or tear string 62 may be replaced with a side tab that is used to break the perforations 66 around the individual package. Yet in other embodiments, packages may not have tear strips or tabs and are simply opened by tearing open the package at either side edge 54 or upper edge 52.

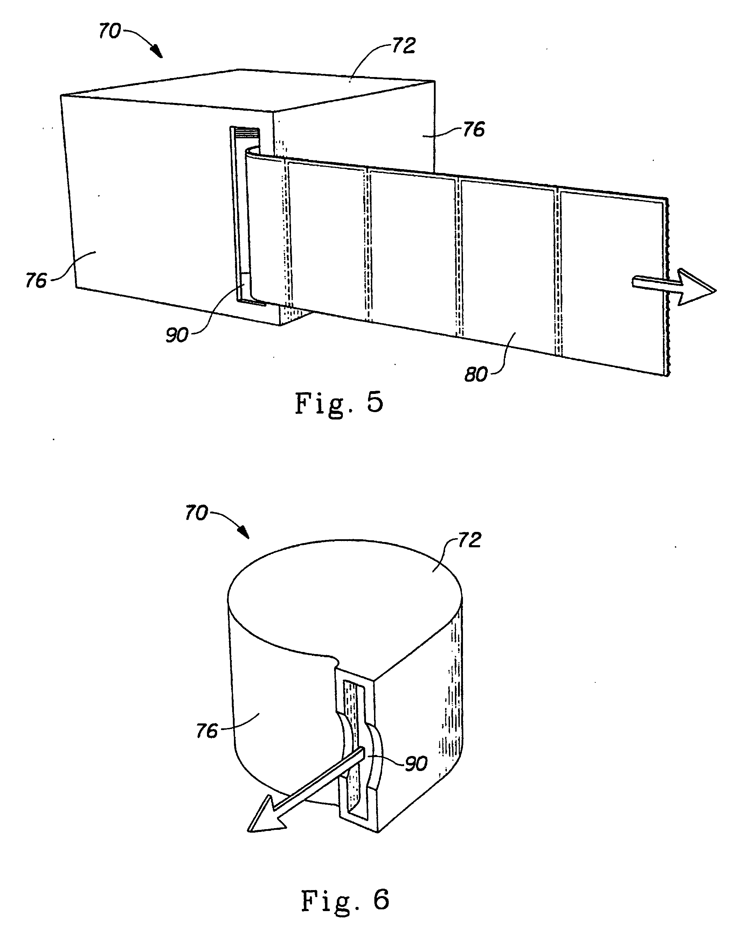

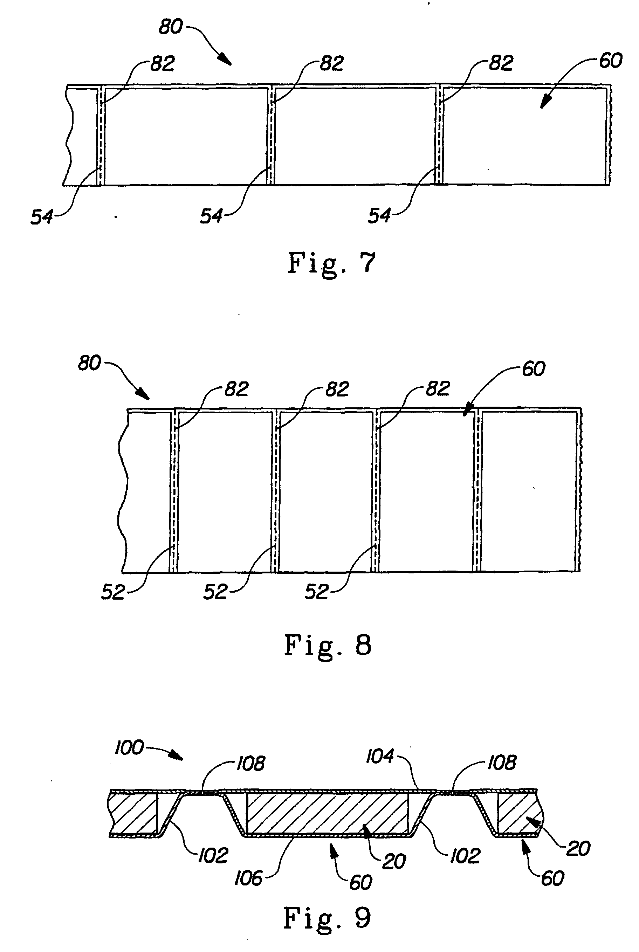

The individual package 60 of FIG. 4 may be packaged and stored according to an embodiment of the present invention shown in FIG. 5. The package for a plurality of absorbent interlabial products (or case) 70 contains a plurality of individually packaged absorbent interlabial devices 80 and is used to store and dispense the individual packages 60 individually or in a group. Preferably, the individual packages 60 are connected in a continuous series with each other. The series of individual packages may be connected along their side edges 54 (as shown in FIG. 7) or along their longitudinal edges 52 (as shown in FIG. 8). The case 70 may contain 2 to 100 individually packaged absorbent interlabial devices, preferably may contain 2 to 48 individually packaged absorbent interlabial devices, more preferably may contain 2 to 24 individually packaged absorbent interlabial devices, which can be separated into smaller units containing 1 or more individually packaged absorbent interlabial devices.

The upper longitudinal edge 52 and side edges 54 are preferably frangibly sealed together forming crimped edges 56 to close off the sides and ends of the package to form a separate inner compartment to protect the absorbent interlabial products. Suitable methods for frangibly sealing the edges of a package are described in U.S. Patent 4,556,146 issued to Swanson, et al., U.S. Patent 5,181,610 issued to Quick, and U.S. Patent 5,462,166 issued to Minton, et al. However, instead of being separated during the manufacturing process, the packages can be left connected. Preferably, there are perforations 82 separating each individually packaged interlabial device 60 in the series of individual packages to aid in separation from the succeeding package.

The case 70 can be in any suitable configuation. One embodiment of a case 70 is shown in FIG. 5. The case 70 shown in FIG. 5 has an upper wall 72, and a lower wall 74. In this embodiment, the upper wall 72 and lower wall 74 each have an outer perimeter that is generally square or rectangular. The four side walls 76 are oriented orthogonally and longitudinally between the upper wall 72 and the lower wall 74 so that the side walls 76 are attached along the outer perimeter of both the upper wall 72 and the lower wall 74, oppositely connecting the upper wall 72 and the lower wall 74 to form an enclosed shape. The case 70 has an opening to form a dispenser, from which the packages are dispensed from the interior of the case 70, on either upper wall 72, lower wall 74 or side wall 76. The case 70 is generally rectangular, however in an alternative embodiment shown in FIG. 6, the side wall 76 can be oriented circularly along the outer perimeters of the upper wall 72 and the lower wall 74 creating a cylindrical shape for the case 70.

FIG. 9 shows an alternative embodiment for packaging an interlabial device. In the embodiment shown in FIG. 9, the interlabial devices are contained in a strip of blister packages. The blister packages define individual compartments 100 for the interlabial devices 20. The interlabial device 20 may be packaged in the compartment 100 as shown, or wrapped in an individual wrapper 50 first and then packaged in the individual compartment 100. The individual compartment 100 comprises a continuous side wall 102, a top wall 104 that is adjacent to and in one embodiment, removably attached around the periphery of the side wall 102. The juncture 108 of the side wall 102 and top wall 104 is frangibly connected to a preceding compartment 100 at or adjacent to the juncture 108 of the preceding side wall 102 and top wall 104. The compartment 100 also comprises a bottom wall 106 that is joined to the side wall 102 create a cup-shaped cavity that holds the interlabial device 20 or the individually packaged interlabial device 60. Either the interlabial device 20 or the individually packaged interlabial device 60 is removed from the compartment 100 when the top wall 104 is removed, or broken to remove the interlabial device 20.

The compartments 100 may be then packaged in the case 70 and be dispensed individually or in multiples. The case 70 may contain 2 to 100 compartments 100, preferably may contain 2 to 64 compartments 100, more preferably may contain 2 to 36 compartments 100, which can be separated into smaller units containing 1 or more compartments 100.

## Claims

1. A dispenser with a plurality of absorbent interlabial products (20) comprising:
a continuous strip of spaced absorbent interlabial products (20) comprising two or more packages (60) for said absorbent interlabial products (20), said packages (60) have seals that form an individual and separate inner compartment to protect said absorbent interlabial products (20) from contaminants, and said packages (60) are joined together by a frangible connection located in between said seals to provide a line of weakness that when broken separates said packages (60) into individual packages (60) of absorbent interlabial products (20); and,
a case (70) for dispensing said packages (60), wherein said case (70) having an interior from which said packages (60) are dispensed.

2. The dispenser of Claim 1 wherein said line of weakness connection comprises at least a perforation line that is positioned after at least a portion of each seal for the individual package (60).

3. The dispenser of Claim 1 wherein said package (60) comprises an individual compartment (100), said compartment (100) comprising a continuous side wall (102), a top wall (104) adjacent to and removably attached around the periphery of said side wall (102), wherein said top wall (104) of said compartment (100) is frangibly connected to the top wall (104) of a proceeding package (60), and a bottom wall (106), wherein said bottom wall (106) and said side wall (102) create a cup-shaped cavity that holds said absorbent interlabial product (20), whereby said absorbent interlabial product (20) is removed from said package (60) when said bottom wall (106) is removed.

4. The dispenser of Claim 3 wherein said interlabial product (20) is individually wrapped inside said package (60).

5. The dispenser of Claim 1 having a continuous strip of connected products (20) containing 2 to 100 packages (60) which can be separated into smaller units.

6. The dispenser of Claim 1 having a continuous strip of connected products (20) containing 2 to 48 packages (60) which can be separated into smaller units.

7. The dispenser of Claim 1 having a continuous strip of connected products (20) containing 2 to 24 packages (60) which can be separated into smaller units.

8. The dispenser of Claim 1 having a continuous strip of connected products (20) containing 2 to 12 packages (60) which can be easily separated into smaller units.

9. The dispenser of Claim 1 having a continuous strip of connected products (20) wherein said product (20) is oriented horizontally.

10. The dispenser of Claim 1 having a continuous strip of connected products (20) wherein said product (20) is oriented vertically.

11. The dispenser of Claim 1, wherein said
case (70) has an upper wall (72), and a lower wall (74), said upper wall (72) and lower wall (74) each having an outer perimeter and at least one side wall (76) oriented longitudinally between said upper wall (72) and said lower wall (74) wherein said side wall (76) is attached along the outer perimeter of both said upper wall (72) and said lower wall (74) oppositely connecting said upper (72) and said lower wall (74) to form an enclosed shape, wherein said side wall (76) has an opening (90) to form a dispenser from which said packages (60) are dispensed from the interior of said case (70).

12. The dispenser of Claim 11 wherein said outer perimeter is generally square or rectangular, and comprises four side walls (76) oriented orthogonally and longitudinally between said upper wall (72) and said lower wall (74) wherein said side walls (76) are attached along the outer perimeter of both said upper wall (72) and said lower wall (74) oppositely connecting said upper (72) and said lower wall (74) to form an enclosed shape, wherein said side wall (76) has an opening (90) to form a dispenser from which said packages (60) are dispensed from said interior of said case (70).

13. The dispenser of Claim 11 wherein said case (70) is generally cylindrical and said side wall (76) is oriented circularly around said upper wall (72) and said lower wall (74).

## Patentansprüche

1. Spender mit einer Mehrzahl von absorbierenden Interlabial-Produkten (20) mit:
einem kontinuierlichen Streifen von in Abstand zueinander liegenden absorbierenden Interlabial-Produkten (20) mit zwei oder mehreren Verpackungen (60) für die absorbierenden Interlabial-Produkte (20), wobei die Verpackungen (60) Dichtungen haben, die eine einzelne und separate innere Abteilung bilden, um die absorbierenden Interlabial-Produkte (20) vor Verunreinigungen zu schützen, und wobei die Verpackungen (60) miteinander durch eine zerbrechliche Verbindung verbunden sind, die zwischen den Dichtungen liegt, so daß sie eine Schwächungslinie bilden, die, wenn sie zerbrochen ist, die Verpackungen (60) in einzelne Verpackungen (60) der absorbierenden Interlabial-Produkte (20) separiert; und
einem Gehäuse (70) zum Ausgeben der Verpackungen (60), wobei das Gehäuse (70) ein Inneres hat, von welchem aus die Verpackungen (60) ausgegeben werden.

2. Spender nach Anspruch 1, in welchem die Schwächungslinien-Verbindung wenigstens eine Perforationslinie umfaßt, die nach wenigstens einem Bereich jeder Dichtung für die einzelne Verpackung (60) positioniert ist.

3. Spender nach Anspruch 1, in welchem die Verpackung (60) eine einzelne Abteilung (100) aufweist, wobei die Abteilung (100) aufweist eine kontinuierliche Seitenwand (102), eine Oberseitenwand (104), die an den Umfang der Seitenwand (102) angrenzt und daran herum abnehmbar angebracht ist, wobei die Oberseitenwand (104) der Abteilung (100) mit der Oberseitenwand (104) einer vorausgehenden Verpackung (60) und einer Bodenwand (106) zerbrechlich verbunden ist, wobei die Bodenwand (106) und die Seitenwand (102) einen tassenförmigen Hohlraum erzeugen, der das absorbierende Interlabial-Produkt (20) hält, wobei das absorbierende Interlabial-Produkt (20) aus der Verpackung (60) entnommen wird, wenn die Bodenwand (106) entfernt ist.

4. Spender nach Anspruch 3, in welchem das Interlabial-Produkt (20) innerhalb der Verpackung (60) einzeln eingehüllt ist.

5. Spender nach Anspruch 1, mit einem kontinuierlichen Streifen von verbundenen Produkten (20), der 2 bis 100 Verpackungen (60) enthält, welche in kleinere Einheiten separiert werden können.

6. Spender nach Anspruch 1, mit einem kontinuierlichen Streifen von verbundenen Produkten (20), die 2 bis 48 Verpackungen (60) enthalten, welche in kleinere Einheiten separiert werden können.

7. Spender nach Anspruch 1, mit einem kontinuierlichen Streifen von verbundenen Produkten (20), die 2 bis 24 Verpackungen (60) enthalten, die in kleinere Einheiten separiert werden können.

8. Spender nach Anspruch 1, mit einem kontinuierlichen Streifen aus verbundenen Produkten (20), die 2 bis 12 Verpackungen (60) enthalten, die leicht in kleinere Einheiten separiert werden können.

9. Spender nach Anspruch 1, mit einem kontinuierlichen Streifen aus verbundenen Produkten (20), wobei das Produkt (20) horizontal ausgerichtet ist.

10. Spender nach Anspruch 1, mit einem kontinuierlichen Streifen aus verbundenen Produkten (20), wobei das Produkt (20) vertikal ausgerichtet ist.

11. Spender nach Anspruch 1, in welchem das Gehäuse (70) eine obere Wand (72) und eine untere Wand (74) hat, wobei die obere Wand (72) und die untere Wand (74) jeweils einen äußeren Umfang und jeweils eine Seitenwand (76), die in Längsrichtung zwischen der oberen Wand (72) und der unteren Wand (76) ausgerichtet ist, haben, wobei die Seitenwand (76) entlang des äußeren Umfangs sowohl der oberen Wand (72) als auch der unteren Wand (74) angebracht ist und die obere (72) und die untere Wand (76) gegenüberliegend verbindet, um eine geschlossene Gestalt zu bilden, wobei die Seitenwand (76) eine Öffnung (80) hat, um einen Spender zu bilden, aus welchem die Verpackungen (60) aus dem Inneren des Gehäuses (70) ausgegeben werden.

12. Spender nach Anspruch 11, in welchem der äußere Umfang im wesentlichen quadratisch oder rechtwinklig ist und welcher vier Seitenwände (76) aufweist, die rechtwinklig und längs zwischen der oberen Wand (72) und der unteren Wand (74) ausgerichtet sind, wobei die Seitenwände (76) entlang des äußeren Umfangs sowohl der oberen Wand (72) als auch der unteren Wand (74) angebracht sind und die obere (72) und die untere Wand (74) gegenüberliegend verbinden, um eine geschlossene Gestalt zu bilden, wobei die Seitenwand (76) eine Öffnung (90) hat, um einen Spender zu bilden, aus welchem die Verpackungen (60) aus dem Inneren des Gehäuses (70) ausgegeben werden.

13. Spender nach Anspruch 11, in welchem das Gehäuse (70) zylindrisch ist und die Seitenwand (76) kreisförmig um die obere Wand (72) und die untere Wand (74) herum ausgerichtet ist.

## Revendications

1. Distributeur comportant une pluralité de produits absorbants interlabiaux (20) comprenant :
une bande continue de produits absorbants interlabiaux (20) espacés comprenant deux ou plus de deux conditionnements (60) destinés auxdits produits absorbants interlabiaux (20), lesdits conditionnements (60) comportant des joints d'étanchéité qui forment un compartiment intérieur individuel et séparé afin de protéger lesdits produits absorbants interlabiaux (20) de souillures, et lesdits conditionnements (60) sont réunis ensemble par une liaison fragile située entre lesdits joints d'étanchéité afin de fournir une ligne de faiblesse qui, lorsqu'elle est rompue, sépare lesdits conditionnements (60) en conditionnements individuels (60) de produits absorbants interlabiaux (20) ; et
un boîtier (70) destiné à distribuer lesdits conditionnements (60), dans lequel ledit boîtier (70) comporte une partie intérieure à partir de laquelle lesdits conditionnements (60) sont distribués.

2. Distributeur selon la revendication 1, dans lequel ladite liaison à ligne de faiblesse comprend au moins une ligne de perforation qui est placée après au moins une partie de chaque joint d'étanchéité destinée au conditionnement individuel (60).

3. Distributeur selon la revendication 1, dans lequel ledit conditionnement (60) comprend un compartiment (100) individuel, ledit compartiment (100) comprenant une paroi latérale continue (102), une paroi de dessus (104) adjacente et fixée de manière amovible à la périphérie de ladite paroi latérale (102), dans lequel ladite paroi de dessus (104) dudit compartiment (100) est reliée de manière fragile à la paroi de dessus (104) d'un conditionnement (60) suivant, et une paroi de fond (106), dans lequel ladite paroi de fond (106) et ladite paroi latérale (102) engendrent une cavité en forme de coupelle qui maintient ledit produit absorbant interlabial (20), de sorte que ledit produit absorbant interlabial (20) est retiré dudit conditionnement (60) lorsque ladite paroi de fond (106) est retirée.

4. Distributeur selon la revendication 3, dans lequel ledit produit interlabial (20) est enveloppé individuellement à l'intérieur dudit conditionnement (60).

5. Distributeur selon la revendication 1, comportant une bande continue de produits (20) reliés contenant 2 à 100 conditionnements (60) qui peuvent être séparés en unités plus petites.

6. Distributeur selon la revendication 1, comportant une bande continue de produits (20) reliés contenant 2 à 48 conditionnements (60) qui peuvent être séparés en unités plus petites.

7. Distributeur selon la revendication 1, comportant une bande continue de produits (20) reliés contenant 2 à 24 conditionnements (60) qui peuvent être séparés en unités plus petites.

8. Distributeur selon la revendication 1, comportant une bande continue de produits (20) reliés contenant 2 à 12 conditionnements (60) qui peuvent être séparés facilement en unités plus petites.

9. Distributeur selon la revendication 1, comportant une bande continue de produits (20) reliés, dans lequel ledit produit (20) est orienté horizontalement.

10. Distributeur selon la revendication 1, comportant une bande continue de produits (20) reliés, dans lequel ledit produit (20) est orienté verticalement.

11. Distributeur selon la revendication 1, dans lequel ledit boîtier (70) présente une paroi supérieure (72) et une paroi inférieure (74), lesdites paroi supérieure (72) et paroi inférieure (74) ayant chacune un périmètre extérieur et au moins une paroi latérale (76) orientée longitudinalement entre ladite paroi supérieure (72) et ladite paroi inférieure (74), dans lequel ladite paroi latérale (76) est fixée le long du périmètre extérieur, à la fois, de ladite paroi supérieure (72) et de ladite paroi inférieure (74), reliant de manière opposée lesdites paroi supérieure (72) et paroi inférieure (74) afin de former une forme fermée, dans lequel ladite paroi latérale (76) présente une ouverture (90) afin de former un distributeur à partir duquel lesdits conditionnements (60) sont distribués de l'intérieur dudit boîtier (70).

12. Distributeur selon la revendication 11, dans lequel ledit périmètre extérieur est globalement carré ou rectangulaire et comprend quatre parois latérales (76) orientées orthogonalement et longitudinalement entre ladite paroi supérieure (72) et ladite paroi inférieure (74), dans lequel lesdites parois latérales (76) sont fixées le long du périmètre extérieur, à la fois, de ladite paroi supérieure (72) et de ladite paroi inférieure (74), reliant de manière opposée lesdites paroi supérieure (72) et paroi inférieure (74) afin de former une forme fermée, dans lequel ladite paroi latérale (76) présente une ouverture (90) afin de former un distributeur à partir duquel lesdits conditionnements (60) sont distribués de l'intérieur dudit boîtier (70).

13. Distributeur selon la revendication 11, dans lequel ledit boîtier (70) est globalement cylindrique et ladite paroi latérale (76) est orientée de manière circulaire autour de ladite paroi supérieure (72) et de ladite paroi inférieure (74).
